Europäisches Patentamt

European Patent Office

Office européen des brevets

(11) Publication number: **0 382 451**

**A2**

(12) **EUROPEAN PATENT APPLICATION**

(21) Application number: 90301181.5

(22) Date of filing: 05.02.90

(51) Int. Cl.⁵: **C07K 7/10, A61K 37/02**

(30) Priority: **07.02.89 US 307642**

(43) Date of publication of application:
**16.08.90 Bulletin 90/33**

(84) Designated Contracting States:
**CH DE FR GB IT LI NL**

(71) Applicant: **MERCK & CO. INC.**
**126, East Lincoln Avenue P.O. Box 2000**
**Rahway New Jersey 07065-0900(US)**

Applicant: **TEMPLE UNIVERSITY**

**Philadelphia Pennsylvania 19122(US)**

(72) Inventor: **Friedman, Paul A.**
**403 Great Spring Road**
**Rosemont, PA 19010(US)**
Inventor: **Jacobs, John W.**
**298 W. Court St.**
**Doylestown, PA 18901(US)**
Inventor: **Gould, Robert J.**
**973 Gravel Pike**
**Green Lane, PA 18054(US)**

Inventor: **Polokoff, Mark A.**
**29 Phyllis Place**
**Randolf, NJ 07869(US)**
Inventor: **Gan, Zhong-Ru**
**Pembrook Garden, Apt. 8-F**
**North Wales, PA 19454(US)**
Inventor: **Niewiarowski, Stefan**
**Apartment 26, 90 445 Woodbine Avenue**
**Narberth, PA 19072(US)**
Inventor: **Holt, John C.**
**323 Moreland St.**
**Philadelphia , PA 19198(US)**
Inventor: **Rucinski, Boguslaw**
**12 E. Newfield Way**
**Bala-Cynwyd, PA 19004(US)**
Inventor: **Williams, Janice A.**
**4213 Regents Square, Apt. 2**
**Philadelphia, PA 19104(US)**

(74) Representative: **Hesketh, Alan, Dr.**
**European Patent Department Merck & Co.,**
**Inc. Terlings Park Eastwick Road**
**Harlow Essex, CM20 2QR(GB)**

(54) Viper venom Polypeptides and variants.

(57) A platelet aggregation inhibiting polypeptide having the following amino acid sequence:
X-Cys-R-R-R-Arg-Gly-Asp-R-R-R-R-R-Cys-Y
wherein X is H or at least one amino acid; Y is OH or at least one amino acid; and each R, either the same or different, is any amino acid.

Polypeptides having this sequence are potent inhibitors of fibrinogen binding to receptors expressed on the glycoprotein IIb/IIIa complex in the membrane of platelets, and are therefore potent inhibitors of fibrinogen-induced human platelet aggregation. They are useful in inhibition of platelet aggregation formation.

# VIPER VENOM POLYPEPTIDES AND VARIANTS

FIELD OF THE INVENTION

The invention relates to polypeptides for inhibiting platelet aggregation.

BACKGROUND OF THE INVENTION

This invention relates generally to modulating cell adhesion and to inhibiting the binding of fibrinogen and other proteins to blood platelets, and inhibiting the aggregation of blood platelets. Fibrinogen is a glycoprotein, present in blood plasma, which participates in platelet aggregation and in fibrin formation. Platelets are cell-like anucleated fragments, found in the blood of all mammals, which participate in blood coagulation. Interaction of fibrinogen with a receptor, the platelet membrane glycoprotein complex IIb/IIIa, is known to be essential for normal platelet function.

When a blood vessel is damaged, platelets adhere to the disrupted subendothelial surface. The adherent platelets subsequently release biologically active constituents and aggregate. Aggregation is initiated by the binding of agonists, such as thrombin, epinephrine, or ADP to specific platelet membrane receptors. Stimulation by agonists results in exposure of latent fibrinogen receptors on the platelet surface, and binding of fibrinogen to the glycoprotein IIb/IIIa complex.

Attempts have been made to use natural products and synthetic peptides to study the mechanism of platelet aggregation and adhesion. Platelet aggregation inhibitor proteins have been isolated from a variety of viper venoms. However, studies of the mechanism of inhibition of platelet aggregation by these proteins have been hampered by the lack of structural information.

Recently, a venom peptide called trigramin was characterized in some detail (see Huang et al., J. of Biol. Chem., 1987, 262, pp. 16157-16163 and U.S. Serial No. 165,661, filed March 8, 1988). The peptide was reported to competitively inhibit fibrinogen binding to the glycoprotein IIb/IIIa complex on the platelet membrane. The peptide contains an Arg-Gly-Asp sequence which is believed to be close to the COOH-terminal residue.

Ruslahti and Pierschbacher, Science, 1987, 238, pp. 491-497, describe adhesive proteins such as fibronectin, vitronectin, osteopontin, collagens, thrombospondin, fibrinogen, and von Willebrand factor present in extracellular matrices and in the blood. The proteins contain the tripeptide arginine-glycine-aspartic acid as their cell recognition site. The tripeptides are recognized by at least one member of a family of structurally related receptors, integrins, which are heterodimeric proteins with two membrane-spanning subunits. The authors state that the conformation of the tripeptide sequence in the individual proteins may be critical to recognition specificity.

Cheresh, Proc. Natl. Acad. Sci. USA, 1987, 84, pp. 6471-6475, describes an Arg-Gly-Asp directed adhesion receptor expressed by human endothelial cells that is structurally similar to the IIb/IIIa complex on platelets but antigenically and functionally distinct. The receptor is directly involved in endothelial cell attachment to fibrinogen, von Willebrand factor, and vitronectin.

Pierschbacher and Ruslahti, J. of Biol. Chem., 1987, 262, 36, pp. 17294-17298 describe influence of stereochemistry of the sequence Arg-Gly-Asp-Xaa on binding specificity of peptides containing the tripeptide sequence Arg-Gly-Asp. In Proc. Nat'l. Acad. Sci. USA, 1984, 81, pp. 5985-5988, the same authors describe variants of the cell recognition site of fibronectin that retain attachment-promoting activity. The tetrapeptide Arg-Gly-Asp-Ser is described as the minimal structure recognized by cells in the large, adhesive glycoprotein fibronectin. Peptides having portions -Arg-Gly-Asp-Ser- are described in U.S. Patent Nos. 4,589,881 and 4,614,517. Peptides having portions -Arg-Gly-Asp-R wherein R is selected from Thr or Cys or other amino acid having the same cell-attachment activity as fibronectin, are described in U.S. Patent No. 4,578,079.

Ruggeri et al., Proc. Nat'l. Acad. Sci. USA, 1986, 83, pp. 5708-5712, describes a series of synthetic peptides, designed in lengths to 16 residues, that contain the sequence Arg-Gly-Asp-Val, which inhibit fibrinogen binding to platelets.

Gold et al., Circulation, 77, 3, March 1988, pp. 670-677 describes the effects of bolus injections of recombinant single-chain tissue-type plasminogen activator and of F(ab')$_2$ fragments of a murine monoclonal antibody (7E3) against the human platelet GPIIb/IIIa receptor on coronary arterial thrombolysis and reocclusion. Gold et al. found that injection of F(ab')$_2$ fragments of the monoclonal antibody 7E3, directed

EP 0 382 451 A2

against the platelet GPIIb/IIIa receptor, accelerates thrombolysis with recombinant single-chain tissue-type plasminogen activator and prevents reocclusion.

While it is known that the tripeptide sequence Arg-Gly-Asp is present in certain polypeptides which can duplicate or inhibit the cell attachment-promoting effects of fibronectin and vitronectin, there is little understanding of the influence on binding specificity of other amino acids in the polypeptide. Applicants have identified and purified polypeptides which contain the tripeptide sequence Arg-Gly-Asp, which are platelet aggregation inhibitors and which have cysteine residues specifically positioned in relation to the position of the tripeptide. The specific location of the cysteine amino acids appears to significantly influence the ability of these polypeptides to inhibit platelet aggregation.

SUMMARY OF THE INVENTION

The present invention comprises polypeptides characterized in that they inhibit fibrinogen-platelet binding and have the general formula I:

X-Cys-R-R-R-Arg-Gly-Asp-R-R-R-R-R-Cys-Y      (I)

wherein X is H or at least one amino acid; Y is OH or at least one amino acid; and each R, either the same or different, is any amino acid.

Polypeptides within the formula I include those of formula Ia:

H₂N-(Ch)-Cys-R-R-R-Arg-Gly-Asp-R-R-R-R-R-Cys-(Cx)-H      (Ia)

wherein Ch represents at least one amino acid; Cx represents at least one amino acid; and each R, either the same or different, represents an amino acid.

Polypeptides within the formula Ia further include the polypeptides of formula Ib:

H₂N-(Cb)-Cys-R-Cys-R-R-R-R-R-R-Cys-Cys-R-R-R-R-Cys-R-R-R-R-R-R-Cys-R-R-R-R-Cys-Cys-R-R-Cys-R-R-R-R-R-R-R-R-Cys-R-R-R-Arg-Gly-Asp-R-R-R-R-R-Cys-R-R-R-R-R-R-Cys-(Cz)-H      (Ib)

wherein Cb represents at least one amino acid; Cz represents at least one amino acid; and each R, same or different, is an amino acid.

Excluded from the scope of the present invention, as defined by formula Ib, is the compound known as trigramin, which has the following amino acid sequence:

Glu-Ala-Gly-Glu-Asp-Cys-Asp-Cys-Gly-Ser-Pro-Ala-Asn-Pro-Cys-Cys-Asp-Ala-Ala-Thr-Cys-Lys-Leu-Ile-Pro-Gly-Ala-Gln-Cys-Gly-Glu-Gly-Leu-Cys-Cys-Asp-Gln-Cys-Ser-Phe-Ile-Glu-Glu-Gly-Thr-Val-Cys-Arg-Ile-Ala-Arg-Gly-Asp-Asp-Leu-Asp-Tyr-Cys-Asn-Gly-Arg-Ser-Ala-Gly-Cys-Pro-Arg-Asn-Pro-Phe-His

Polypeptides according to formula Ib wherein the amino acid immediately following the Arg-Gly-Asp sequence is Phe, are particularly active inhibitors of platelet aggregation.

Polypeptides within the formula Ib most preferably include the polypeptides of formula Ic:

H₂N-(Cc)-Gly-Glu-R-Cys-Asp-Cys-Gly-R-Pro-R-Asn-Pro-Cys-Cys-Asp-Ala-Ala-Thr-Cys-Lys-Leu-R-Pro-Gly-Ala-Gln-Cys-R-R-Gly-Leu-Cys-Cys-Asp-Gln-Cys-R-Phe-R-R-R-R-R-Ile-Cys-Arg-R-Ala-Arg-Gly-Asp-R-R-Asp-AsP-R-Cys-Thr-Gly-R-Ser-Ala-R-Cys-Pro-Arg-(Cw)-H      (Ic)

wherein Cc represents zero, one or two amino acids; Cw represents zero, one, two, three or four amino acids; and each R, same or different, is an amino acid.

Polypeptides of the invention have been purified from the venom of various vipers, e.g. Trimeresurus gramineus, Echis carinatus, Aqkistrodon piscivorus, Bitis arietans, Trimeresurus elegans, Trimeresurus albolabris and Bothrops atrox. These polypeptides are useful for inhibiting fibrinogen binding to human platelets and inhibiting fibrinogen-induced aggregation of human platelets.

These polypeptides are purified from viper venom according to the purification procedure described below. They are rich in cysteine and contain the common sequence -Cys-R-R-R-Arg-Gly-Asp-R-R-R-R-R-Cys-, where each R, either the same or different, represents any amino acid.

Although Applicants do not wish to be bound to any particular theory regarding binding mechanism, it is believed that the location of the cysteine amino acids plays an important role in determining the three-dimensional conformation, degree of rigidity and binding specificity of the polypeptides.

DETAILED DESCRIPTION OF THE INVENTION

Polypeptides within the scope of the present invention have been isolated from viper venom of Trimeresurus gramineus, Echis carinatus and Agkistrodon piscivorus, Bitis arietans, Trimeresurus elegans, Trimeresurus albolabris, Bothrops atrox and Trimeresurus flavoridis. All of these polypeptides are potent platelet aggregation inhibitors.

3

EP 0 382 451 A2

The sequence for an inhibitor isolated from Echis carinatus venom is:

Glu-Cys-Glu-Ser-Gly-Pro-Cys-Cys-Arg-Asn-Cys-Lys-Phe-Leu-Lys-Glu-Gly-Thr-Ile-Cys-Lys-Arg-Ala-Arg-Gly-Asp-Asp-Met-Asp-Asp-Tyr-Cys-Asn-Gly-Lys-Thr-Cys-Asp-Cys-Pro-Arg-Asn-Pro-His-Lys-Gly-Pro-Ala-Thr

This inhibitor is hereinafter referred to as Echistatin.

The sequence for an inhibitor isolated from Agkistrodon piscivorus venom is:

Val-Gln-Pro-Ala-Asn-Pro-Cys-Cys-Asp-Ala-Ala-ThrCys-Lys-Leu-Thr-Pro-Gly-Ser-Gln-Cys-Ala-Glu-Gly-Leu-Cys-Cys-Asp-Gln-Cys-Lys-Phe-Ile-Lys-Ala-Gly-???-Ile-Cys-???-Arg-Ala-Arg-Gly-Asp-Asn...

The identities of certain amino acids are not known at this time, nor is the identity of the sequence following the 46th amino acid. The inhibitor is nevertheless believed to fall within the scope of the present invention, and is hereinafter identified as Agkistrostatin.

One sequence for an inhibitor isolated from Bitis arietans venom is:

Ser-Pro-Pro-Val-Cys-Gly-Asn-Glu-Leu-Leu-Glu-Glu-Gly-Glu-Glu-Cys-Asp-Cys-Gly-Ser-Pro-Ala-Asn-Cys-Gln-Asp-Arg-Cys-Cys-Asn-Ala-Ala-Thr-Cys-Lys-Leu-Thr-Pro-Gly-Ser-Gln-Cys-Asn-His-Gly-Glu-Cys-Cys-Asp-Gln-Cys-Lys-Phe-Lys-Lys-Ala-Arg-Thr-Val-Cys-Arg-Ile-Ala-Arg-Gly-Asp-Trp-Asn-Asp-Asp-Tyr-Cys-Thr-Gly-Lys-Ser-Ser-Asp-Cys-Pro-Trp-Asn-His

The inhibitor is hereinafter identified as Bitistatin 1.

Another sequence for an inhibitor isolated from Bitis arietans venom is:

Ser-Pro-Pro-Val-Cys-Gly-Asn-Lys-Ile-Leu-Glu-Gln-Gly-Glu-Asp-Cys-Asp-Cys-Gly-Ser-Pro-Ala-Asn-Cys-Gln-Asp-Gln-Cys-Cys-Asn-Ala-Ala-Thr-Cys-Lys-Leu-Thr-Pro-Gly-Ser-Gln-Cys-Asn-His-Gly-Glu-Cys-Cys-Asp-Gln-Cys-Lys-Phe-Lys-Lys-Ala-Arg-Thr-Val-Cys-Arg-Ile-Ala-Arg-Gly-Asp-Trp-Asn-Asp-Asp-Tyr-Cys-Thr-Gly-Lys-Ser-Ser-Asp-Cys-Pro-Trp-Asn-His

The inhibitor is hereinafter identified as Bitistatin 2.

Another sequence for an inhibitor isolated from Bitis arietans venom is:

Val-Ser-Pro-Pro-Val-Cys-Gly-Asn-Lys-Ile-Leu-Glu-Gln-Gly-Glu-Asp-Cys-Asp-Cys-Gly-Ser-Pro-Ala-Asn-Cys-Gln-Asp-Gln-Cys-Cys-Asn-Ala-Ala-Thr-Cys-Lys-Leu-Thr-Pro-Gly-Ser-Gln-Cys-Asn-His-Gly-Glu-Cys-Cys-Asp-Gln-Cys-Lys-Phe-Lys-Lys-Ala-Arg-Thr-Val-Cys-Arg-Ile-Ala-Arg-Gly-Asp-Trp-Asn-AsP-Asp-Tyr-Cys-Thr-Gly-Lys-Ser-Ser-Asp-Cys-Pro-Trp-Asn-His

The inhibitor is hereinafter identified as Bitistatin 3.

The sequence for an inhibitor isolated from Trimeresurus elegans venom is:

Gly-Glu-Glu-Cys-Asp-Cys-Gly-Ser-Pro-Glu-Asn-Pro-Cys-Cys-Asp-Ala-Ala-Thr-Cys-Lys-Leu-Arg-Pro-Gly-Ala-Gln-Cys-Ala-Asp-Gly-Leu-Cys-Cys-Asp-Gln-Cys-Arg-Phe-Lys-(?)-(?)-Arg-Thr-Ile-Cys-Arg-Arg-Ala-Arg-Gly-Asp-Asn-Pro-Asp-Asp-Arg-Cys-Thr-Gly-Gln-Ser-Ala-Asp-Cys-Pro-Arg-Asn-Gly-Leu-Tyr

The inhibitor is nevertheless believed to fall within the scope of the present invention, and is hereinafter identified as Elegantin.

The sequence for an inhibitor isolated from Trimeresurus albolabris venom is:

Glu-Ala-Gly-Glu-Asp-Cys-Asp-Cys-Gly-Ser-Pro-Ala-Asn-Pro-Cys-Cys-Asp-Ala-Ala-Thr-Cys-Lys-Leu-Leu-Pro-Gly-Ala-Gln-Cys-Gly-Glu-Gly-Leu-Cys-Cys-Asp-Gln-Cys-Ser-Phe-Met-Lys-Lys-Gly-Thr-Ile-Cys-Arg-Arg-Ala-Arg-Gly-Asp-Asp-Leu-Asp-Asp-Tyr-Cys-Asn-Gly-Ile-Ser-Ala-Gly-Cys-Pro-Arg-Asn-Pro-Leu-His-Ala

The inhibitor is hereinafter identified as Albolabrin.

The sequence for an inhibitor isolated from Bothrops atrox venom is:

Glu-Ala-Gly-Glu-Glu-Cys-Asp-Cys-Gly-Thr-Pro-Glu-Asn-Pro-Cys-Cys-Asp-Ala-Ala-Thr-Cys-Lys-Leu-Arg-Pro-Gly-Ala-Gln-Cys-Ala-Glu-Gly-Leu-Cys-Cys-Asp-Gln-Cys-Arg-Phe-Lys-Gly-Ala-Gly-Lys-Ile-Cys-Arg-Arg-Ala-Arg-Gly-Asp-Asn-Pro-Asp-Asp-Arg-Cys-Thr-Gly-Gln-Ser-Ala-Asp-Cys-Pro-Arg-Asn-Arg-Phe

The inhibitor is hereinafter identified as Batroxostatin.

The sequence for an inhibitor isolated from Trimeresurus flavoridis venom is:

Gly-Gly-Glu-Cys-Asp-Cys-Gly-Ser-Pro-Glu-Asn-Pro-Cys-Cys-Asp-Ala-Ala-Thr-Cys-Lys-Leu-Arg-Pro-Gly-Ala-Gln-Cys-Ala-Asp-Gly-Leu-Cys-Cys-Asp-Gln-Cys-Arg-Phe-Lys-(?)-(?)-Arg-Thr-Ile-Cys-Arg-Ile-Ala-Arg-Gly-Asp-Phe-Pro-Asp-Asp-Arg-Cys-Thr-Gly-Leu-Ser-Ala-Asp-Cys-Pro-Arg-(?)-Asn-Asp-Leu

The identities of three amino acids are not known at this time. The inhibitor is nevertheless believed to fall within the scope of the present invention, and is hereinafter identified as flavoridin.

The sequences identified above are specific examples of polypeptides falling within the sequence defined in formula I, and should not be interpreted as limiting the scope of the present invention.

EXAMPLE 1

4

METHODS

Materials

- The venom of Echis carinatus was purchased from Miami Serpentarium Laboratories, Salt Lake City, UT. Low molecular weight peptide standards, Sephadex G-50, and Mono S FPLC columns were from Pharmacia, Inc. Servalyt Precote isoelectric focusing gels were purchased from Serva Fine Biochemicals, Inc., and isoelectric focusing marker proteins were from BDH Chemicals, Ltd. C18 HPLC columns were the products of Vydac.

Preparation of Platelets

Rabbit whole blood was collected from New Zealand white rabbits. Blood was centrifuged at 200 x g for 10 min. The supernatant, platelet rich plasma, was centrifuged for 15 min at 800 x g in the presence of apyrase (20 $\mu$g/ml) and PGE1 (5 ng/ml). The pellet was resuspended in 10 ml of wash buffer (138 mM NacL, 2.9 mM KCl, 0.31 mM $Na_2HPO_4$, 1 mM $MgCl_2$, 5 mM HEPES, pH 6.5, 5 mM glucose, 0.1% $NaHCO_3$, 1 mM EGTA and 0.35% bovine serum albumin). Apyrase was added to a final concentration of 20 $\mu$g/ml. The resuspended pellet was centrifuged at 800 x g for 15 min, then washed a second time in the same buffer. The washed platelets were then suspended in wash buffer, pH 7.4, with EGTA omitted at a concentration of 2-5 x $10^8$ cells/ml.

Platelet Aggregation Assay

Platelet aggregation was measured at 37C in an aggregometer. The reaction mixture contained washed platelets (2-5 x $10^8$ cells/ml), fibrinogen (100 $\mu$g/ml), $Ca^{2+}$ (1 mM), ADP (10 $\mu$M), epinephrine (2 $\mu$g/ml), and the platelet aggregation inhibitor fraction to be tested. The aggregation was initiated by adding ADP and epinephrine 1 min after the other components had been added. The reaction was allowed to proceed for at least 2 min. The extent of inhibition of aggregation was expressed as the percentage of aggregation observed in the absence of inhibitor. In some experiments platelet aggregation was studied in platelet rich plasma upon addition of 10 $\mu$M ADP.

Purification of Echistatin

Rabbit platelet aggregation assays were used to monitor the activity during purification. Echis carinatus lyophilized venom (1g) was dissolved in 12 ml of 10 mM ammonium bicarbonate, pH 7.7 containing 20 mM DTT. After 15 min at room temperature, the solution was centrifuged at 45,000 x g for 45 min. The pellet was discarded and the supernatant was loaded onto a 2.5 x 125 cm column of Sephadex G-50 equilibrated and eluted at 4° with 10 mM MES, pH 5.3. Fractions containing platelet aggregation inhibitory activity were pooled and concentrated under vacuum. After desalting on a column of Sephadex G-15, 10-13 mg of protein were loaded onto a Mono S FPLC column which had been equilibrated with 10 mM MES, pH 5.3. The bound protein was fractionated at 4° with a linear gradient from 0 to 0.3 M NaCl to 10 mM MES, pH 5.3. The flow rate was 0.6 ml/min. Inhibitory activity eluted as a single peak at about 0.17 M NaCl. The pooled activity was loaded directly onto a C18 reverse phase HPLC column which had been equilibrated with 0.1% (v/v) trifluoroacetic acid in water. The column was developed at room temperature with a polyphasic acetonitrile gradient in 0.1% aqueous TFA, at a flow rate of 0.7 ml/min. Inhibitory activity eluted as a single peak at an acetonitrile concentration of 17%. Volatile material was removed by vacuum centrifugation followed by lyophilization. The resulting protein was homogeneous as judged by SDS polyacrylamide gel electrophoresis, isoelectric focusing, rechromatography on reverse phase HPLC, and N-terminal sequence determination. The yield of purified protein, which we have named Echistatin, was 2.2 mg from 1 g. of starting material.

SDS Polyacrylamide Gel Electrophoresis

5

We used a modification of the Laemmli system (Nature. 1970, 227, pp. 680-685, hereby incorporated by reference). Stacking and separating gels (1.5 mm) contained 8% and 20% polyacrylamide, respectively. Urea (8 M) was added to the separating gel. The gels were run at 85 volts for 1.5 hr and then at 90 volts for 17 hr. Protein was detected by Coomassie brilliant blue R-250 staining.

Isoelectric Focusing

A Servalyt Precote isoelectric focusing gel was used. Samples were run at a constant power of 1 W in an LKB Ultraphore apparatus. The gels were stained with Serva Blue W.

Reduced and Carboxymethylated Echistatin

Echistatin (0.75 mg protein) was reduced for 1 hr at room temperature with 20 mM DTT in the presence of 0.28 M Tris and 6.7 M guanidine hydrochloride, in a volume of 0.6 ml. Iodoacetic acid (0.06 ml) was then added to give a final concentration of 0.136 M and alkylation was allowed to proceed for 20 min at room temperature. The reduced, carboxymethylated Echistatin was isolated from reagents by C18 reverse phase HPLC.

Chymotryptic Cleavage of Echistatin

Echistatin was digested with chymostrypsin at a ratio of substrate to protease of 40:1 (w/w). The proteolytic reaction was carried out for 4 hr at 37C in 0.2 M ammonium bicarbonate, pH 7.8. The reaction mixture was then lyophilized, and chymotryptic peptides were isolated by reverse phase HPLC.

Amino Acid Sequence Determination

Purified Echistatin and selected chymotryptic fragments were subjected to automated protein sequence analysis in an Applied Biosystems 470A Sequencer using the sequencer program and reagents supplied by the manufacturer. Released amino acid derivatives were identified with the aid of an on-line HPLC system.

Protein Determination

Protein was determined by the method of Lowry et al. (J. of Biol. Chem., 1951, 193, pp. 265-275, hereby incorporated by reference) using bovine serum albumin as standard.

RESULTS

Purification

Echistatin was purified to homogeneity from the soluble portion of the lyophilized venom of Echis carinatus in three chromatographic steps. Due to the presence of platelet aggregation stimulatory activity in the crude venom, the inhibitor could not be reliably detected in this fraction. Following gel filtration on Sepahdex G-50, however, a single peak of inhibitory activity was observed. This activity was further purified by cation exchange FPLC on Mono S, and finally by C18 reverse phase HPLC.

One gram of lyophilized venom yielded 2.2 mg of purified Echistatin. This material exhibited a single band on SDS polyacrylamide gel electrophoresis in the presence of 8M urea and on isoelectric focusing. Rechromatography on reverse phase HPLC resulted in a single symmetrical absorbance peak which corresponded exactly with platelet aggregation inhibitory activity. Further confirmation of the homogeneity of the Echistatin preparation was obtained during amino acid sequencing of the native or the reduced and carboxymethylated protein. The only amino acid residue observed during the first sequencer cycle was

6

glutamic acid.

## Molecular Weight and Isoelectric Point

The apparent molecular weight of reduced Echistatin during electrophoresis on 20% SDS polyacrylamide gels in the presence of 8 M urea was 5,800 daltons. This is similar to the value calculated from the amino acid sequence (see below). Under nonreducing conditions, the protein migrated somewhat more slowly, corresponding to an apparent molecular weight of 7,000 daltons. Surprisingly, when native Echistatin was treated with hydroxyethyl disulfide, the apparent molecular weight was only about 4,000 daltons. A minor band, probably resulting from incomplete derivatization, was observed in the hydroxyethyl disulfide-treated Echistatin.

Both native and disulfide-treated Echistatin exhibited on pI of 8.3. Upon treatment with dithiothreitol, a minor band with a pI of 7.5 was detected in addition to the predominant band at pI 8.3.

## Amino Acid Composition

The amino acid composition of Echistatin, obtained after 20 hr of hydrolysis of the reduced, carboxymethylated protein, is shown in Table I. The composition obtained by this analysis was compared with that predicted from the sequence (see below). Most values obtained by these two methods agreed closely. The content of cysteinyl residues found by amino acid composition (6.7) was lower than that predicted by sequence (8). This may have been due to incomplete carboxymethylation of Echistatin.

TABLE I

| Comparison of the amino acid composition of Echistatin between the value determined after hydrolysis and that calculated from sequence: | | |
|---|---|---|
| Amino Acid | Residues per molecule | |
| | Acid hydrolysis | Sequence |
| Cysteine | 6.5 | 8 |
| Aspartic acid 1 | 8.4 | 8 |
| Threonine | 2.8 | 3 |
| Serine | 1.2 | 1 |
| Glutamic acid 2 | 3.3 | 3 |
| Proline | 4.2 | 4 |
| Glycine | 5.3 | 5 |
| Alanine | 1.8 | 2 |
| Methionine | 0.6 | 1 |
| Isoleucine | 1.0 | 1 |
| Leucine | 1.1 | 1 |
| Tyrosine | 1.0 | 1 |
| Phenylalanine | 1.0 | 1 |
| Histidine | 1.2 | 1 |
| Lysine | 5.2 | 5 |
| Arginine | 4.1 | 4 |
| Total | 48.7 | 49 |

[1]The value includes asparagine.
[2]The value includes glutamine.

Effect of Echistatin on platelet aggregation in vitro

Platelet aggregation studies indicate that Echistatin is more potent than trigramin in inhibiting human gel filtered platelet aggregation induced by ADP in the presence of 0.1 M mg/ml fibrinogen ($IC_{50} = 30$ nM vs. 150 nM).

Echistatin inhibits aggregation of human platelets induced by ADP collagen, platelet activating factor or epinephrine at doses 30-300 nM.

Human Platelet Rich Plasma

Blood was drawn into 0.1 vol. of 3.8% sodium citrate by venipuncture from normal human volunteers. Platelet rich plasma (PRP) was prepared by centrifugation at 400 x g for 12 min. Aggregations were performed by adding ADP collagen, platelet activating factor or epinephrine to PRP and measured in a Sienco aggregometer.

Human Gel Filtered Platelets

Blood was drawn into 0.1 volume of acid-citrate-dextrose (85 mM sodium citrate, 64 mM citric acid, 110 mM dextrose) by venipuncture from normal human volunteers. Platelet rich plasma was prepared by centrifugation at 400 x g for 12 min. $PGE_1$ (5 μg/ml) was added and Platelets were collected by centrifugation at 800 x g for 12 min. The platelet pellet was resuspended into human platelet buffer (140 mM NaCl, 7.9 mM KCl, 3.2 mM $Na_2HPO_4$, 6 mM HEPES, 2% bovine serum albumin, 0.1% dextrose, pH 7.2) and filtered over Sepharose 2B that was previously equilibrated in human platelet buffer. Platelets were counted and adjusted to $2 \times 10^8$/ml with human platelet buffer.

Amino Acid Sequence

The amino acid sequence of Echistatin was obtained by automated Edman degradation of the reduced, carboxymethylated protein. The protein has 49 amino acids with an N-terminal glutamic acid residue and a C-terminal threonine, and is shown above. Cysteine comprised 8 residues, or greater than 16%, of the total amino acids in Echistatin. The sequence was repeated two times with the same result. When native Echistatin was sequenced, no PTH-amino acid peaks were observed during sequencer cycles where cysteinyl residues were expected, further confirming the assignments at these residues. The disulfide status of these cysteinyl residues is not known.

Further confirmation of the sequence was obtained by analysis of chymotryptic peptides isolated from the reduced, carboxymethylated protein (Table II).

TABLE II

| Amino Acid Composition of Chymotryptic Peptides of Echistatin | | | | | |
|---|---|---|---|---|---|
| The values in parentheses are the numbers of amino acid residues determined by sequence. | | | | | |
| | C1 | C2 | C3 | C4 | C3 + C4 |
| Cys (CM) | 2.6(4) | 0.7(1) | 1.3(3) | | 1.5(3) |
| Asx | 1.3(1) | | 2.7(3) | | 2.9(3) |
| Thr | | 1.0(1) | 1.0(1) | 1.0(1) | 1.4(2) |
| Ser | 1.1(1) | | | | |
| Glu | 2.0(2) | 1.1(1) | | | |
| Gly | 1.2(1) | 1.4(1) | 1.9(1) | 1.2(1) | 2.2(2) |
| Ala | | | | 1.0(1) | 0.8(1) |
| Ile | | 0.6(1) | | | |
| Leu | | 0.9(1) | | | |
| His | | | 1.2(1) | | 1.3(1) |
| Phe | 0.9(1) | | | | |
| Lys | 1.1(1) | 1.9(2) | 1.7(1) | 1.0(1) | 2.2(2) |
| Arg | 1.2(1) | 0.7(1) | 1.1(1) | | 1.1(1) |
| Pro | 1.3(1) | | 1.4(2) | 1.0(1) | 2.5(3) |

Echistatin contains the sequence Arg-Gly-Asp which is common to certain proteins which bind to the glycoprotein IIb/IIIa complex on the platelet surface, at residues 24-26. This sequence is part of a putative platelet binding site on the fibrinogen molecule.

Echistatin exhibited unusual behavior during SDS polyacrylamide gel electrophoresis in the presence of 8M urea. The native protein migrated with an apparent molecular weight of 7,000 daltons, compared with the value of 5,400 daltons obtained from sequence analysis. Under nonreducing conditions, proteins with intrachain disulfide bonds generally bind less than expected amounts of SDS, while prior reduction increases SDS binding to levels seen with proteins lacking intrachain disulfide bonds (Pitt-Rivers et al., Biochem J., 1968, 109, pp. 825-830). Thus the increased mobility of reduced Echistatin during SDS polyacrylamide gel electrophoresis suggests that this protein contains one or more intrachain disulfide bonds in its native conformation.

Treatment of Echistatin with 80 mM dithiothreitol at room temperature for 20 min completely destroyed its inhibitory activity towards platelet aggregation. Under similar conditions, 80 mM hydroxyethyl disulfide treatment led to the loss of 50% of the inhibitory activity. These results, like those of the SDS gel electrophoresis studies, imply that one or more intrachain disulfide bonds play an important role in maintaining the conformation of Echistatin necessary for its inhibitory activity.

## EXAMPLE 2

Methods of protein purification and determination used in Example 1 were employed to isolate and identify a platelet aggregation inhibitor present in Agkistrodon piscivorus venom. The protein, whose amino acid sequence is identified above, displayed characteristics of a platelet aggregation inhibitor.

## EXAMPLE 3

Methods of protein purification and determination used in Example 1 were employed to isolate and identify a platelet aggregation inhibitor present in Bitis arietans venom. The protein, whose amino acid sequence is identified above, displayed characteristics of a platelet aggregation inhibitor.

EXAMPLE 4

Methods of protein purification and determination used in Example 1 were employed to isolate and identify a second platelet aggregation inhibitor present in Bitis arietans venom. The protein, whose amino acid sequence is identified above, displayed characteristics of a platelet aggregation inhibitor.

EXAMPLE 5

Methods of protein purification and determination used in Example 1 were employed to isolate and identify a third platelet aggregation inhibitor present in Bitis arietans venom. The protein, whose amino acid sequence is identified above, displayed characteristics of a platelet aggregation inhibitor.

EXAMPLE 6

30-40 mg of lyophilized Trimeresurus elegans venom was dissolved in 500 μl 0.1% trifluoroacetic acid. The resulting suspension was spun to remove the precipitate. The clear supernatant was subjected to reverse phase high performance liquid chromatography in a 2.50 x 4.6 mm chromatography column containing a wide-pore C-18 silica matrix (e.g. Vidac TPRP, The Separation Group, Hesperia, CA). Fractions were eluted with a 0-30% acetylonitrile gradient containing 0.1% trifluoroacetic acid over 45 minutes. The active fraction was identified by means of studying platelet aggregation in platelet-rich plasma, and freeze-dried. The freeze-dried active fraction was redissolved in 300 μl of 0.1% trifluoroacetic acid, applied to the same column, and eluted using acetonitrile gradient containing 0.1% trifluoroacetic acid. In a typical experiment, 0-1.5% gradient was applied for 5 minutes, 15-20% gradient for 25 minutes, and 20-30% gradient for 35 minutes. The active fraction eluted at 21% of acetylonitrile, representing a substantially pure protein.

EXAMPLE 7

The methods of protein purification used in Example 6 was employed to isolate and identify a platelet aggregation inhibitor present in Trimeresurus albolaboris venom. The protein, whose amino acid sequence is identified above, displayed characteristics of a platelet aggregation inhibitor. The active fraction was eluted at 29% of acetylonitrile.

EXAMPLE 8

The methods of protein purification used in Example 6 was employed to isolate and identify a platelet aggregation inhibitor present in Bothrops atrox venom. The protein, whose amino acid sequence is identified above, displayed characteristics of a platelet aggregation inhibitor. The active fraction was eluted at 25% of acetylonitrile.

EXAMPLE 9

The method of protein purification used in Example 6 was employed to isolate and identify a platelet aggregation inhibitor present in Trimeresurus flavoridis venom. The active fraction was eluted at 28% of acetylonitrile. The protein, whose amino acid sequence is identified above, displayed characteristics of a platelet aggregation inhibitor.

Platelet aggregation analysis showed that polypeptides of the present invention significantly inhibited human platelet aggregation.

TABLE III

| EFFECT OF VIPER VENOM POLYPEPTIDES ON INHIBITION OF HUMAN PLATELET AGGREGATION | |
| --- | --- |
| Polypeptide Name | $IC_{50}$ ($\mu$M)(95% Confid. Lim.) |
| Echistatin | 0.032 (0.026-0.039) |
| Elegantin | 0.100 |
| Albolabrin | 0.100 |
| Batroxostatin | 0.060 |
| Flavoridin | 0.006 |

Purification of various polypeptides within formula I to chemical homogeneity according to the present invention has permitted amino acid sequencing of the molecules. It is contemplated that these polypeptides may also be prepared through genetic engineering techniques. Thus, based upon the amino acid sequences disclosed herein, one may advantageously prepare a synthetic gene corresponding to a disclosed amino acid sequence and introduce that gene into an appropriate host by appropriate cloning vectors. Alternatively, it is contemplated that the pure polypeptide may be prepared by obtaining the natural gene from venom producing cells, followed by recombination and cloning. It is therefore understood that the scope of the invention is not merely limited to polypeptides isolated by following the chromatographic procedures disclosed herein, but also includes these polypeptides as they may be prepared by genetic engineering techniques.

Furthermore, polypeptides of the invention may be prepared using solid phase synthesis, such as that described by Merrifield, J. Am. Chem. Soc., 85, 2149 (1964), although other equivalent chemical syntheses known in the art can also be used, such as the synthesis of Houghten, Proc. Natl. Acad. Sci., 82, 5132 (1985). Solid-phase synthesis is commenced from the C-terminus of the peptide by coupling a protected amino acid to a suitable resin, as generally set forth in U.S. Patent No. 4,244,946, issued January 21, 1982 to Rivier et al., the disclosure of which is incorporated herein by reference. Examples of synthesis of this general type are set forth in U.S. Patent Nos. 4,305,872 and 4,316,891. Discussion of the solid-phase synthesis of 41-residue polypeptide is set forth in Science, 213, 1394-1397 (September 1981) in an article by Vale et al., which refers to a more detailed discussion of the synthesis, which appears in an article by Marke et al. in J. Am. Chem. Soc., 103, 3178 (1981).

In synthesizing the polypeptides, the carboxyl terminal amino acid, having its alpha-amino group suitably protected is coupled to a chloro-methylated polystyrene resin or the like. After removal of the alpha-amino protecting group, as by using trifluoroacetic acid in methylene chloride, the next step in the synthesis is ready to proceed. Other standard cleaving reagents and conditions for the removal of specific amino protecting groups may be used, as described in the open literature.

The remaining alpha-amino- and side-chain-protected amino acids are then coupled stepwise in the desired order to obtain an intermediate compound connected to the resin. As an alternative to adding each amino acid separately in the synthesis, some of them may be coupled to one another prior to the addition to the growing solid-phase chain. The selection of the appropriate coupling reagents is within the skill of the art.

Common to chemical synthesis of peptides is the protection of the labile side-chain groups of the various amino acid moieties with suitable protecting groups at that site until the group is ultimately removed after the chain has been completely assembled. Also common is the protection of the alpha-amino group on an amino acid or a fragment while that entity reacts at the carboxyl group, followed by the selective removal of the alpha-amino-protecting group to allow subsequent reaction to take place at that location. Accordingly, it is common that, as a step in the synthesis, an intermediate compound is produced which includes each of the amino acid residues located in the desired sequence in the peptide chain with various of these residues having side-chain protecting groups. These protecting groups are then commonly removed substantially at the same time so as to produce the desired resultant product following purification.

After the desired amino acid sequence has been completed, the intermediate peptide is removed from the resin support by treatment with a reagent, such as liquid HF, which not only cleaves the peptide from the resin, but also cleaves all the remaining side-chain-protecting groups. The polypeptide can then be purified by gel permeation followed by semipreparative HPLC, as described in Rivier et al., Peptides: Structure and Biological Function (1979) pp. 125-128. A purity of at least 93% or higher (based upon all peptides present) is reasonably obtainable and is preferred for clinical testing and/or use. Purity of 98% is practical; however, for certain in vitro applications, lower purity may be acceptable. Accordingly, the polypeptide is considered useful when it is in substantially pure form which, for purposes of this application, means at least about 50 weight percent, based upon all peptides present.

Polypeptides of the invention may be administered in any situation where inhibition of human or mammalian platelet aggregation or adhesion is desired.

Polypeptides of the invention are eliminated from circulation rapidly and are particularly useful in inhibiting platelet aggregation in situations where a strong antithrombotic of short duration of effectiveness is needed. Thus, they may find utility in surgery on peripheral arteries (arterial grafts, carotid endarterectomy) and in cardiovascular surgery where manipulation of arteries and organs, and/or the interaction of platelets with artificial surfaces, leads to platelet aggregation and consumption. The aggregated platelets may form thrombi and thromboemboli. Polypeptides of the invention may be administered to these surgical patients to prevent the formation of thrombi and thromboemboli.

Extracorporeal circulation is routinely used for cardiovascular surgery in order to oxygenate blood. Platelets adhere to surfaces of the extracorporeal circuit. Adhesion is dependent on the interaction between GPIlb/IIIa on the platelet membranes and fibrinogen adsorbed to the surface of the circuit. (Gluszko et al., Amer. J. Physiol., 1987, 252:H, pp. 615-621). Platelets released from artificial surfaces show impaired hemostatic function. Polypeptides of the invention may be administered to prevent adhesion.

Other applications of these polypeptides include prevention of platelet thrombosis, thromboembolism and reocclusion during and after thrombolytic therapy and prevention of platelet thrombosis, thromboembolism and reocclusion after angioplasty of coronary and other arteries and after coronary artery bypass procedures. In many clinical centers patients subjected to these procedures are already receiving antiplatelet drugs which are weaker inhibitors of platelet aggregation as compared to polypeptides of the present invention. Polypeptides of the invention may also be used to prevent myocardial infarction.

These polypeptides may be administered by any convenient means which will result in its delivery into the blood stream in substantial amount. They may be combined with thrombolytic agents such as plasminogen activators or streptokinase in order to inhibit platelet aggregation. They may also be combined with anticoagulants such as heparin, aspirin or warfarin. Intravenous administration is presently contemplated as the preferred administration route. They are soluble in water, and may therefore be effectively administered in solution.

The present invention also includes a composition comprising recombinant single-chain tissue-type plasminogen activator or streptokinase and a polypeptide having a partial sequence -Cys-R-R-R-Arg-Gly-Asp-R-R-R-R-R-Cys-, where each R, either the same or different, represents an amino acid. The invention also includes a method for promoting thrombolysis and preventing reocclusion in a patient which comprises administering to the patient an amount of recombinant single-chain tissue-type plasminogen activator or streptokinase and an amount of a peptide having a sequence -Cys-R-R-R-Arg-Gly-Asp-R-R-R-R-R-Cys-, where each R, either the same or different, represents any amino acid.

In one exemplary application, a suitable amount of Echistatin is intravenously administered to a heart attack victim undergoing angioplasty. Administration occurs during or several minutes prior to angioplasty, and is in an amount sufficient to inhibit platelet aggregation, e.g. an amount which achieves a steady state plasma concentration of between about 0.05 - 2 $\mu$M. Should the patient need to undergo bypass surgery, Echistatin administration may be stopped and will not cause complications during surgery that would be caused by other materials such as plasminogen activators, the effects of which last hours after cessation of administration.

The present invention may be embodied in other specific forms without departing from the spirit or essential attributes thereof, and accordingly, reference should be made to the appended claims, rather than to the foregoing specification, as indicating the scope of the invention.

## Claims

1. A substantially pure polypeptide called "Elegantin" having the following amino acid sequence:
Gly-Glu-Glu-Cys-Asp-Cys-Gly-Ser-Pro-Glu-Asn-Pro-Cys-Cys-Asp-Ala-Ala-Thr-Cys-Lys-Leu-Arg-Pro-Gly-Ala-Gln-Cys-Ala-Asp-Gly-Leu-Cys-Cys-Asp-Gln-Cys-Arg-Phe-Lys-R-R-Arg-Thr-Ile-Cys-Arg-Arg-Ala-Arg-Gly-Asp-Asn-Pro-Asp-Asp-Arg-Cys-Thr-Gly-Gln-Ser-Ala-Asp-Cys-Pro-Arg-Asn-Gly-Leu-Tyr
wherein either R, either the same or different, is any amino acid.

2. A substantially pure polypeptide called "Albolabrin" having the following amino acid sequence:
Glu-Ala-Gly-Glu-Asp-Cys-Asp-Cys-Gly-Ser-Pro-Ala-Asn-Pro-Cys-Cys-Asp-Ala-Ala-Thr-Cys-Lys-Leu-Leu-Pro-Gly-Ala-Gln-Cys-Gly-Glu-Gly-Leu-Cys-Cys-Asp-Gln-Cys-Ser-Phe-Met-Lys-Lys-Gly-Thr-Ile-Cys-Arg-Arg-Ala-Arg-Gly-Asp-Asp-Leu-Asp-Asp-Tyr-Cys-Asn-Gly-Ile-Ser-Ala-Gly-Cys-Pro-Arg-Asn-Pro-Leu-His-Ala

3. A substantially pure polypeptide called "Batroxostatin" having the following amino acid sequence:
Glu-Ala-Gly-Glu-Glu-Cys-Asp-Cys-Gly-Thr-Pro-Glu-Asn-Pro-Cys-Cys-Asp-Ala-Ala-Thr-Cys-Lys-Leu-Arg-Pro-Gly-Ala-Gln-Cys-Ala-Glu-Gly-Leu-CYs-Cys-Asp-Gln-Cys-Arg-Phe-Lys-Gly-Ala-Gly-Lys-Ile-Cys-Arg-Arg-Ala-Arg-Gly-Asp-Asn-Pro-Asp-Asp-Arg-Cys-Thr-Gly-Gln-Ser-Ala-Asp-Cys-Pro-Arg-Asn-Arg-Phe

4. A substantially pure polypeptide called "Flavoridin" having the following amino acid sequence:
Gly-Gly-Glu-Cys-Asp-Cys-Gly-Ser-Pro-Glu-Asn-Pro-Cys-Cys-Asp-Ala-Ala-Thr-Cys-Lys-Leu-Arg-Pro-Gly-Ala-Gln-Cys-Ala-Asp-Gly-Leu-Cys-Cys-Asp-Gln-Cys-Arg-Phe-Lys-R-R-Arg-Thr-Ile-Cys-Arg-Ile-Ala-Arg-Gly-Asp-Phe-Pro-Asp-Asp-Arg-Cys-Thr-Gly-Leu-Ser-Ala-Asp-Cys-Pro-Arg-R-Asn-Asp-Leu
wherein each R, either the same or different, is any amino acid.

5. A method of inhibiting fibrinogen-induced aggregation of mammalian platelets, comprising incubating mammalian platelets with a polypeptide according to any one of Claims 1 to 4.

6. The use of a polypeptide as claimed in any one of Claims 1 to 4 for the preparation of a medicament useful for inhibiting aggregation or adhesion of platelets in a mammal.

7. A method for preparing the purified polypeptide as claimed in any one of Claims 1 - 4 comprising:
(a) dissolving lyophilized snake venom in slightly acidic solution;
(b) centrifuging the solution to obtain supernatant;
(c) loading supernatant onto a column; and
(d) determining fractions containing platelet aggregation inhibitory activity and concentrating under vacuum.